# EUROPEAN PATENT SPECIFICATION

(11) **EP 2 822 396 B1**
(45) Date of publication and mention of the grant of the patent: **09.08.2017**
(21) Application number: 13710910.4
(22) Date of filing: 08.03.2013
(51) Int. Cl.: A23K 20/126, A23K 40/30, A23K 50/10, A23K 50/30, A61P 1/12, A61K 31/366

(54) **ANIMAL FEED COMPOSITION CONTAINING ENCAPSULATED GLUCONO DELTA-LACTONE**
TIERFUTTERZUSAMMENSETZUNG MIT VERKAPSELTEM GLUCONO-DELTA-LACTON
COMPOSITION D'ALIMENT POUR ANIMAUX CONTENANT DU GLUCONO-DELTA-LACTONE ENCAPSULÉ

(30) Priority: 09.03.2012 EP 12158832
(43) Date of publication of application: 14.01.2015
(73) Proprietor: FrieslandCampina Nederland B.V., 3818 LE Amersfoort (NL)
(72) Inventor: MAAS, Johannes Antonius Maria, NL-5412 NK Gemert (NL); HANENBERG, Martinus Johannes Antonius, NL-5469 VP Erp (NL)
(74) Representative: Nederlandsch Octrooibureau
(86) International application number: PCT/NL2013/050152
(87) International publication number: WO 2013/133713

(56) References cited:
- EP-A1- 1 354 520
- FR-A1- 2 782 608
- US-A- 5 008 118
- US-A- 5 605 697
- US-B1- 6 217 915
- US-B1- 6 718 910
- RUGALA H W: "ENCAPSULATION CONTROLS GLD RELEASE - PRODUCTION EFFICIENCY IMPROVED", FOOD PRODUCT DEVELOPMENT, MAGAZINES FOR INDUSTRY, NEW YORK, US, vol. 12, no. 10, 1 January 1978 (1978-01-01), page 28,31, XP008056955, ISSN: 0015-654X

## Description

### TECHNICAL FIELD OF THE INVENTION

The present invention related to an animal feed composition containing encapsulated glucono delta-lactone (GDL), more precisely GDL encapsulated in a water-resistant matrix. The animal feed composition can suitably be used to feed young calves and piglets as the encapsulated GDL contained therein significantly reduces the incidence of diarrhea in neonatal calves and piglets.

### BACKGROUND OF THE INVENTION

Neonatal calf diarrhea (NCD), also known as calf scours, is a common disease affecting the newborn calf. The most critical period is the approximately first 10 days following birth of the calf. Greatest losses occur when calves are kept in close confinement, where the opportunity for transmission of the causative agents of NCD is enhanced by their build-up in the environment. Furthermore, similar to calves, also new born piglets often suffer from diarrhea. The diarrhea and other clinical signs seen with the disease are caused by the interaction of any of several possible infectious causes and predisposing factors such as lack of colostrum, failure to absorb colostral antibody, poor nutrition and environmental effects. Diarrhea, such as NCD, is a costly disease, with losses estimated to be over $250 million annually and death loss of up to 25% of the U.S. calf crop.

Neonatal calf diarrhea is characterized by diarrhea (scouring), progressive dehydration and death. The neonatal or newborn calf with scours will have a watery yellow, gray or greenish diarrhea containing varying amounts of mucus which may be tinged with blood. Soiling of the hindquarters and tail with the diarrheic feces is common. If the disease is allowed to progress untreated, dehydration and electrolyte (ions of body salts such as sodium, potassium, chloride, and bicarbonate) loss will kill the calf.

The normally solid fecal mass is formed by absorption of water from the liquid intestinal content by the cells lining the large intestine. Diarrhea occurs when the capability of the intestine to absorb fluid is impaired. Interference with this absorptive function of the intestine may occur in two ways. Damage to the cells lining the intestine may result from cell destruction by certain infectious agents, resulting in loss of the digestive and absorptive capability of the intestine as well as inflammation. Other infectious agents produce toxins that cause the cell lining of the intestine to produce fluid rather than absorb it. Diarrhea, dehydration and electrolyte loss occur in both instances and have especially severe effects in the newborn animal.

There are numerous infectious causes of NCD, which may be present either singly or in combination. *E. coli* is a very common and serious bacterial cause of NCD. NCD caused by *E. coli* is called colibacillosis. Several forms of colibacillosis occur with some variation in the symptoms produced. There are no distinctive clinical signs that differentiate scours due to *E. coli* infection from those caused by other infectious agents. Some types of *E. coli* produce toxins that cause the intestine to produce fluid rather than absorb it. Death loss from *E. coli* infection may be high, especially in calves under a week of age. Resistance to *E. coli* infection is acquired by the calf from the colostrum or first milk of the cow.

*Salmonella* infection is another cause of NCD that occurs particularly in confined animals such as dairy calves. Salmonellosis is most severe in calves under a month of age. These organisms are the cause of paratyphoid infection in man and constitute a potential health hazard for people associated with calves affected with salmonellosis.

*Rotavirus* or *Coronavirus* infections is another cause of NCD. Calves 1-7 days of age seem to be most often affected with this disease agent. The disease will appear suddenly and spread rapidly through a calf herd. The virus causes extensive damage to the intestinal lining, resulting in rapid fluid loss and dehydration in calves. Other organisms such as E. coli may infect the calf at the same time.

While usually less common, numerous other infectious causes of NCD exist, including protozoa such as *Cryptosporidia* and *coccidia*, and additional types of bacteria, viruses and virus-like agents. Non-infectious causes may also be important; these include improper diet or feeding practices, or poor quality milk replacer.

It will be understood that reducing the incidence of diarrhea in calves and piglets, in particular reducing the incidence of NCD and/or reducing the mortality rates associated with NCD is highly desirable. Attempts have been made to achieve this by providing animal feed for neonatal calves or piglets that contains an antimicrobial agent or that contains calf-specific micro-organisms.

EP-A 1 354 520 describes a microencapsulated product for animal feeding that comprises a core of n-butyric powder and its salts covered by a lipidic structure constituted by fats, gelatins, polymers, waxes, buffer substances. Butyric acid is said to inhibit the growth of *E. coli*.

WO 2011/120929 describes animal feed for calves comprising calf-specific microorganisms of the genera *Lactococcus*, *Lactobacillus*, *Leuconostoc*, *Enterococcus*, *Streptococcus*, *Propionibacterium*, *Bifidobacterium*, *Eubacterium*, *Pediococcus*, *Veillonella*, *Bacteroides* and/or *Escherichia* as well as optionally lipids, proteins, vitamins and/or mineral materials.

The application of GDL in animal feed is described in US 6,718,910. This US patent describes a method for raising an animal comprising administering to said animal with a feed or drinking water at least one compound selected from the group consisting of: a hexose-derived acid(s), a non-toxic salt(s) thereof and an intramolecular ester compound(s) thereof, wherein said at least one compound is administered to animals in an amount of 20 to 1,300 mg/kg/day and wherein said at least one compound promotes the growth of enterobacteria having the ability to produce a short-chain fatty acid.

US 5,605,697 describes a bifidobacterium growth promotant comprising gluconic acid, a nontoxic salt thereof and/or glucono-delta-lactone as an active ingredient. The bifidobacterium growth promotant has selective bifidobacterial growth promoting-activity and, at the same time, inhibits growth of deleterious bacteria. The bifidobacterium growth promotant can be used per se or as an additive for various foods and drink to provide functional foods and drinks.

### SUMMARY OF THE INVENTION

The inventors have discovered that the incidence of diarrhea in neonatal calves or piglets, in particular NCD, can be reduced significantly by including encapsulated glucono delta-lactone (GDL) in animal feed for neonatal calves or piglets, such as calf milk replacers or sow milk replacer. Accordingly, one aspect of the invention relates to an animal feed composition comprising 0.05-8.0 wt.%, based on the total composition, of GDL-containing encapsulate, wherein the GDL is encapsulated in a water-resistant encapsulation matrix and wherein the composition is a calf milk replacer or sow milk replacer.

GDL (IUPAC name: (3R,4S,5S,6R)-3,4,5-trihydroxy-6-(hydroxymethyl)tetrahydro-2H-pyran-2-one) is a commonly used additive that is used as a sequestrant, an acidifier, or a curing, pickling, or leavening agent. It is a lactone (cyclic ester) of D-gluconic acid. Pure GDL is a white odorless crystalline powder. Upon addition to water, GDL is partially hydrolysed to gluconic acid, with the balance between the lactone form and the acid form established as a chemical equilibrium. The rate of hydrolysis of GDL is increased by heat and high pH.

It is known to use encapsulated in GDL in the production of sausages. Rugala, HW (Encapsulation controls GDL release -production efficiency improved, Food Product Development (1978), 12(10), 28-31) reports that an encapsulated GDL has been developed to provide controlled precise release and controlled pH adjustment in the preparation of fermented sausage products. The encapsulated GDL (Durkote GDL 13S-50) comprised 50% GDL encapsulated with hydrogenated vegetable fat.

Although the inventors do not wish to be bound by theory it is believed that the advantageous effect of ingested encapsulated GDL on the incidence of diarrhea in neonatal calves and piglets, in particular NCD, is associated with the delayed release of gluconic acid in the colon. In order for gluconic acid to exert a favorable effect in the colon it is believed to be important that the gluconic acid is released in the colon rather than in the small intestines or the stomach.

The present invention also relates to the therapeutic or prophylactic treatment of diarrhea in calves and piglets using the aforementioned animal feed composition.

### DETAILED DESCRIPTION OF THE INVENTION

A first aspect of the present invention relates to an animal feed composition comprising 0.05-8.0 wt.%, based on the total composition, of GDL-containing encapsulate, wherein the GDL is encapsulated in a water-resistant encapsulation matrix and wherein the composition is a calf milk replacer or sow milk replacer.

The term "encapsulated GDL" as used herein refers to an encapsulate that comprise one or more particles of GDL embedded in an encapsulation matrix. The encapsulate may, for instance, contain a core particle that comprises GDL and a protective coating that surrounds the core particle. Alternatively, the encapsulate may contain a plurality of GDL-containing particles that are distributed throughout an encapsulation matrix.

The animal food composition according to the present invention preferably is a powder, a paste or a liquid. The encapsulated GDL is preferably homogeneously distributed throughout the composition in admixture with one or more feed components.

In accordance with a preferred embodiment, the animal feed composition contains 0.08-6. wt.%, more preferably 0.1-4.0 wt.% even more preferably 0.2-3.0. wt.%, and most preferably 0.2-2.0 wt % of encapsulated GDL.

The GDL is encapsulated in a water-resistant encapsulation matrix in order to delay the release of GDL during the passage through the gastro-intestinal tract.

The ability of the GDL encapsulate to delay the release of GDL during passage through the gastrointestinal tract can be demonstrated by suspending the encapsulate into aqueous whey protein solution and by monitoring the pH of the water as a function of time (whilst continuously stirring the suspension). This release test is performed as follows:
- prepare a stock solution of 10% (w/v) cheese whey powder in tap water and adjust pH to 7.2 with 1M NaOH;
- prepare 200 ml samples of the stock solution and maintain these samples at 37°C;
- add GDL encapsulate to a sample in an amount equivalent to 0.8% (w/v) GDL;
- measure the pH of the sample at regular intervals.

When non-encapsulated GLD is submitted to the aforementioned release test, pH of the solution decreases to below 5.0 in about 90 minutes. In contrast thereto, the GDL encapsulates according to the present invention typically produce such a pH decrease after not less than 4 hours, even more preferably after not less than 5 hours and most preferably after not less than 6 hours.

Water resistance may be imparted to the GDL encapsulate by encapsulating the GDL in a lipophilic matrix. Preferably, said lipophilic matrix contains 50-100 wt.%, more preferably 70-100 wt.% and most preferably 90-100 wt.% of one or more lipophilic components selected from triglycerides, diglcyerides, monoglycerides, esters of monoglycerides, metal salts of fatty acids, esters of diglycerides, phospholipids, fatty acid esters of sugars and waxes. According to a particularly preferred embodiment, the one or more lipophilic components are selected from triglycerides (e.g. a stearin fraction of a lauric fat), diglycerides, metal salts of fatty acids, for example calcium stearate, and waxes.

According to another preferred embodiment, the lipophilic matrix contains at least 40 wt.%, more preferably at least 60 wt.% and most preferably at least 70 wt.% triglycerides.

The release of GDL may be delayed effectively by employing a lipohilic matrix having a melting point above body temperature. Thus, the lipophilic matrix preferably has a melting point of at least 40°C. Even more preferably, the lipophilic matrix has a melting point of at least 50°C, most preferably of at least 55°C.

The encapsulation matrix typically represents 10-90 wt.% of the encapsulated GDL. More preferably, the encapsulation matrix represents 20-80 wt.%, even more preferably 30-70 wt.% and most preferably 40-60 wt.% of the encapsulated GDL. GDL typically represents 20-90 wt.%, more preferably 30-80 wt.% and most preferably 40-70 wt. of the encapsulated GDL. Together, the encapsulation matrix and GDL typically represent 60-100 wt.%, more preferably 70-100 wt.% and most preferably 80-100 wt.% of the encapsulated GDL.

The release characteristics of the encapsulated GDL may be modified to further delay the release of GDL by e.g. applying multiple coating layers, including a coating layer that is made of an indigestible component (e.g. wax, sucrose fatty acid polyester, non digestible carbohydrates (like ethylcellulose, cellulose, cellulose derivatives, oligofructose, galacto oligosaccharides)). Delayed release may also be achieved by employing an encapsulation matrix that contains a significant amount of one or more indigestible components.

To enhance the water dispersability (lump formation prevention) of the encapsulated GDL, it may additionally be coated or mixed with an oil comprising at least 10 wt.% medium chain triglycerides (i.e. triglycerides comprising 2 or 3 fatty acid residues with 6-12 carbon atoms) and/or with an amphiphilic substance, for instance protein or lecithin. This may be achieved by e.g. fluid bed agglomeration. Alternatively, said oil or the amphiphilic substance may be sprayed on the encapsulated GDL particles. In case lecithin is used, the lecithin may be dissolved or dispersed in a feed or food grade oil and sprayed onto the encapsulated GDL.

The encapsulated GDL typically has a mass weighted average diameter in the range of 200-2000 µm, more preferably of 400-1500 µm and most preferably of 600-1200 µm. Typically, at least 80 wt.% of the encapsulated GDL has a particle size within the range of 200-2000 µm, more preferably within the range of 300-1800 µm and most preferably within the range of 400-1600 µm.

According to a particularly preferred embodiment, the encapsulated GDL has a density similar to the density of water so that the encapsulated GDL will show no tendency to float or sediment when the animal feed is water based or when it is reconstituted with water. Thus, advantageously the encapsulated GDL has a density of 0.8-1.4 g/ml, more preferably of 0.9-1.3 g/ml and most preferably of 0.95-1.2 g/ml.

In order to adjust the density of the encapsulated GDL to approach the density of water, a weighting agent may be incorporated in the encapsulate. Suitable weighting agents include minerals, e.g. milk minerals or milk calcium salts (calcium phosphate, calcium citrate or mixtures) and/or carbohydrates, e.g. UF permeate of milk or whey, delactosed permeate, or lactose. Other weighting agents that can suitably be used include silicon dioxide, titanium dioxide, and clay types like kaolin.

The animal feed composition of the present invention preferably contains 0.05-2.0 wt.% GDL. Even more preferably, the GDL content is 0.1-1.0. wt.%. Most preferably, the composition contains 0.15-0.8 wt.% GDL.

The benefits of the present composition are particularly evident if the composition is used to feed neonatal calves. The present invention may also be used for similar purpose as sow milk replacer for feeding piglets, preferably up to six weeks after birth. For piglets, the composition of the invention can suitably be used in liquid or powder form.

The animal feed composition of the present invention typically comprises, by weight of dry matter:
- 5-30% fat;
- 10-30% protein;
- 20-65% carbohydrate.;
- 2-15 % minerals.

For neonatal calves, the fat content of the composition preferably lies in the range of 10-30%, more preferably 10-28%, most preferably 10-25%, by weight of dry matter. For piglets, the fat content in the animal feed composition is preferably 6-28%, more preferably 8-28%, most preferably 8-26% by weight of dry matter. The protein content preferably lies in the range of 12-28%, most preferably 15-28%, by weight of dry matter. As a protein source, use is preferably made of whey protein concentrate and/or wheat gluten hydrolysate. The carbohydrate content of the feed composition preferably lies in the range of 30-65%, most preferably 35-65%, by weight of dry matter. Preferably, at least 30 wt.%, more preferably at least 40 wt.% and most preferably at least 50 wt.% of the protein contained in the animal feed composition is dairy protein, preferably whey protein.

Lactose preferably represents a substantial fraction of the carbohydrate contained in the animal feed composition. Preferably, lactose represents at least 60 wt.%, most preferably at least 75 wt.% of the carbohydrates contained in the composition.

The animal feed composition of the present invention preferably contains 3-15%, most preferably 5-12% minerals by weight of dry matter. Typically, the composition contains 0.2-2.0%, more preferably 0.3-1.75%, most preferably 0.4-1.5% by weight of dry matter of calcium. Typically, the composition contains 50-200 ppm, more preferably 65-175 ppm, most preferably 75-150 ppm by weight of dry matter of iron.
Other minerals that are preferably contained in the animal feed are:

| | |
|---|---|
| Cu | : 1-25 ppm, preferably 3-20 ppm and most preferably 5-15 ppm; and/or |
| Zn | : 50-250 ppm, preferably 75-225 ppm and most preferably 100-200 ppm; and/or |
| Mn | : 5-50 ppm, preferably 10-45 ppm and most preferably 15-40 ppm; and/or |
| Se | : 0.1-2 ppm, preferably 0.2-1.5 ppm and most preferably 0.3-1.0 ppm; and/or |
| I | : 0.1-5 ppm, preferably 0.2-3 ppm and most preferably 0.5-2 ppm. |

The animal feed composition preferably contains added vitamins. Advantageously, the animal feed per kg contains 10,000-100,000 IU, more preferably 15,000-80,000 IU, most preferably 20,000-60,000 IU of vitamin A. Advantageously, the animal feed per kg contains 1,000-10,000 IU, more preferably 1,500-8,000 IU, most preferably 2,000-6,000 IU of vitamin D, especially vitamin D3.

Other vitamins that are advantageously contained in the animal feed include:

| | |
|---|---|
| Vitamin E | : 20-200 ppm, preferably 40-150 ppm and most preferably 60-100 ppm; |
| Vitamin C | : 25-250 ppm, preferably 50-200 ppm and most preferably 75-175 ppm. |

According to a particularly preferred embodiment, the present animal feed composition is a powder, notably a powder that can be reconstituted with water to produce a calf milk replacer or sow milk replacer. The powder typically has a bulk density of 0.4-1.0 g/ml. Even more preferably, the powder has a bulk density of 0.4-0.8 g/ml. The encapsulated GDL that is employed in the animal feed composition of the present invention may suitably be produced by encapsulation techniques known in the art. Examples of such encapsulation techniques include spray chilling/spray cooling, fluid bed encapsulation, extrusion etc.

Another aspect of the present invention relates to the use of the animal feed composition as described herein before in therapeutic or prophylactic treatment of diarrhea in calves and piglets. Typically, said treatment comprises at least once daily feeding of the calves said feed composition. Preferably, said feeding occurs during a period of a least 7 day during the first 3, preferably first 9 weeks after birth. For piglets, use is intended at least daily in the first 6 weeks after birth.

The invention is further illustrated by the following non-limiting examples.

### EXAMPLES

### Example 1

A GDL encapsulate containing 40 wt.% GDL (Lysactone® PL, Roquette) and 60 wt.% fat (Revel™ A, IOI Group, Loders Croklaan) was prepared by means of spray cooling.

The fat used is a fractionated, non hydrogenated triglyceride fat of lauric origin having the solid fat profile described in Table 1:

**Table 1**

| **Temperature (°C)** | **Solid fat content (%)** |
|---|---|
| 20 | 92-96 |
| 30 | 86-91 |
| 35 | 82-86 |
| 40 | 74-81 |

The encapsulate was produced by the following process:
- In a mixing vessel, 12 kg of the fat was melted at 70°C, and 8 kg of GDL was mixed in and dispersed homogeneously throughout the melted fat. At this point other components like wax or ingredients to increase the weight can optionally be mixed in.
- The melted fat mixture was transported to a spray cool tower (Niro Atomizer), using a homogeniser (Niro Soavi type LS 200611), operated a zero Bar set pressure. Due to back pressure, the operating pressure rose to 50 Bar. The connecting tubing from the homogeniser to the spray cool tower was heated using electric heat tracing in order to prevent solidification of the fat-GDL mix.
- The fat mixture was sprayed via a single nozzle and cooled down by cold (20°C) air inlet. At the point the particles reach the bottom of the atomizer they are solidified and can be collected at the end of the run.

The diameter of the particles in the powder so obtained ranged from 0.2-1.5 mm.

### Example 2

A GDL encapsulate containing 75 wt.% GDL and 25 wt.% fat was prepared with the same materials and process as in Example 1, except that this time 15 kg fat and 5 kg GDL and were used.

### Example 3

The GDL encapsulates described in Example 1 (GDL encapsulate 1, i.e.40wt.% GDL) and Example 2 (GDL encapsulate 2, i.e. 75 wt.% GDL) were applied in an instant calf milk replacer (Kalvoquick™ 1318, FrieslandCampina) at a dosage level of 0.36 wt.% GDL. The instant calf milk replacer had the composition depicted in Table 2.

**Table 2**

| **Ingredients** | **Wt**.**%** |
|---|---|
| Protein enriched whey powder | 78 |
| Vegetable oils and fats (palm, coconut, colza) | 16 |
| Wheat gluten hydrolysate* | 6 |
| | |

| **Composition** | **wt.%** |
|---|---|
| Fat | 16.0 |
| Protein | 21.0 |
| Ash | 9.5 |
| Lactose | 47.5 |
| | |

| **Minerals** | **ppm** |
|---|---|
| Calcium | 5,800 |
| Iron | 100 |
| Copper | 10 |
| Zinc | 140 |
| Manganese | 30 |
| Selene | 0.5 |
| Iodine | 1.1 |
| | |

| **Vitamins** | |
|---|---|
| Vitamin A | 55,000 IU |
| Vitamin D3 | 4,500 IU |
| Vitamin E | 80 ppm |
| Vitamin C | 120 ppm |

| | |
|---|---|
| *SOLPRO available from TATE&LYLE | |

The calf milk replacers were prepared by mixing instant calf milk replacer with water of 45-55° C - in a ratio of 1 kg powder and 7 liters of water - to finally reach a milk replacer solution of 38-40 °C.

Newborn calves were fed with three different calf milk replacers during 8 consecutive weeks:
- CMR1 = calf milk replacer containing GDL encapsulate 1
- CMR2 = calf milk replacer containing GLD encapsulate 2
- Control = calf milk replacer

Over a period of 8 weeks three groups of each 24 calves received 37 kg of one of one of the above calf milk replacers according to a standard feeding scheme. During the first three weeks the calves were housed individually so manure could be collected and analyzed for each individual calf.

The results are described in Table 3.

**Table 3**

| | **CMR1** | **CMR2** | **Control** |
|---|---|---|---|
| Deaths | 0 | 0 | 1 |
| | | | |
| Feed intake (kg/calf) | | | |
| • CMR | • 34.4 | • 34.4 | • 34.4 |
| • Concentrate | • 27.7 | • 28.9 | • 23.0 |
| • Silage maize | • 27.0 | • 28.2 | • 22.3 |
| | | | |
| Weight (kg) | | | |
| • Start | • 42.6 | • 43.0 | • 41.6 |
| • Week 3 | • 52.8 | • 52.8 | • 51.5 |
| • Week 6 | • 69.8 | • 79.7 | • 67.6 |
| • Week 8 | • 81.4 | • 82.7 | • 77.6 |
| | | | |
| Diarrhea (% of calves) | 4 | 5 | 18 |

### Example 4

Example 3 was repeated, except that this time the GDL encapsulates were applied in the calf milk replacer at a dosage of 0.18 wt.% GDL:
- CMR3 = calf milk replacer containing GDL encapsulate 1 (0.18 wt.%)
- CMR4 = calf milk replacer containing GLD encapsulate 2 (0.18 wt.%)
- Control = calf milk replacer

The results are described in Table 4.

**Table 4**

| | **CMR3** | **CMR4** | **Control** |
|---|---|---|---|
| Deaths | 0 | 0 | 0 |
| | | | |
| Feed intake (kg/calf) | | | |
| • CMR | • 36.2 | • 36.1 | • 36.2 |
| • Concentrate | • 27.0 | • 23.4 | • 22.7 |
| • Silage maize | • 26.3 | • 22.6 | • 21.9 |
| | | | |
| Weight (kg) | | | |
| • Start | • 44.3 | • 44.0 | • 44.0 |
| • Week 3 | • 53.5 | • 52.1 | • 52.4 |
| • Week 6 | • 68.4 | • 66.8 | • 66.5 |
| • Week 8 | • 82.6 | • 78.4 | • 78.1 |
| | | | |
| Diarrhea (% of calves) | 9 | 9 | 17 |

### Example 5

The release characteristics of the encapsulate described in Example 1 (40% GDL; 60% fat) were determined as follows:
- a stock solution of 10% (w/v) cheese whey powder in tap water was prepared and pH was adjusted to 7.2 with 1M NaOH;
- 200 ml samples of the stock solution were prepared and maintained at 37°C;
- to one sample non-encapsulated GLD was added in an amount of 0.8% (w/v)
- to another sample the GDL encapsulate was added in an amount of 2.0% (w/v)
- the pH of the samples was measured at regular intervals during 4 hours

The results of this test are summarized in Table 5.

**Table 5**

| **Time (min.)** | **pH** | |
|---|---|---|
| | **Non-encapsulated GDL** | **Encapsulated GDL** |
| 0 | 7,2 | 7,14 |
| 1 | 6,66 | 7,06 |
| 2 | 6,51 | 6,86 |
| 3 | 6,4 | 6,74 |
| 4 | 6,32 | 6,65 |
| 5 | 6,25 | 6,59 |
| 7 | 6,14 | 6,5 |
| 15 | 5,88 | 6,23 |
| 30 | 5,56 | 6,04 |
| 60 | 5,18 | 5,86 |
| 120 | 4,83 | 5,67 |
| 180 | 4,71 | 5,54 |
| 210 | 4,69 | |
| 240 | | 5,43 |

These results show that some GDL is released from the GDL-encapsulate during the test. This is probably GDL that it is present in the encapsulate at or near the surface of the encapsulate particles.

## Claims

1. An animal feed composition comprising 0.05-8.0 wt.%, based on the total composition, of glucono deltalactone (GDL)-containing encapsulate, wherein the GDL is encapsulated in a water-resistant encapsulation matrix and wherein the composition is a calf milk replacer or sow milk replacer.

2. Animal feed composition according to claim 1, wherein the GDL is encapsulated in a lipophilic matrix.

3. Animal feed composition according to claim 2, wherein the lipophilic matrix contains 50-100 wt.% of one or more lipophilic components selected from triglycerides, diglycerides, monoglycerides, esters of monoglycerides, esters of diglycerides, phospholipids, fatty acid esters of sugars and waxes.

4. Animal feed composition according to claim 2 or 3, wherein the lipophilic matrix has a melting point of at least 40°C.

5. Animal feed composition according to any one of the preceding claims, wherein the encapsulated GDL comprises 10-90 wt.% of encapsulation matrix.

6. Animal feed composition according to any one of the preceding claims, wherein GDL represents 20-90 wt.% of the encapsulated GDL.

7. Animal feed composition according to any one of the preceding claims, wherein at least 80 wt.% of the encapsulated GDL has a particle size within the range of 200-2000 µm.

8. Animal feed composition according to any one of the preceding claims, wherein the encapsulated GDL has a density of 0.8-1.4 g/ml.

9. Animal feed composition according to any one of the preceding claims, wherein the feed composition contains 0.05-2.0 wt.% GDL.

10. Animal feed composition according to any one of the preceding claims, wherein the composition comprises, by weight of dry matter:
• 5-30% fat;
• 10-30% protein;
• 20-65% carbohydrate;
• 2-15% minerals.

11. Animal feed composition according to any one of the preceding claims, wherein the composition is a powder.

12. Animal feed composition according to claim 11, wherein the animal feed composition has a bulk density of 0.4-1.0 g/ml.

13. Animal feed composition according to any one of the preceding claims for use in therapeutic or prophylactic treatment of diarrhea in calves or piglets.

## Patentansprüche

1. Tierfutterzusammensetzung, umfassend 0,05-8,0 Gew.-%, bezogen auf die gesamte Zusammensetzung, Glucono-delta-lacton (GDL) enthaltende Verkapselung, wobei das GDL in einer wasserbeständigen Verkapselungsmatrix eingekapselt ist und wobei die Zusammensetzung ein Kälbermilchersatz oder Saumilchersatz ist.

2. Tierfutterzusammensetzung nach Anspruch 1, wobei das GDL in einer lipophilen Matrix eingekapselt ist.

3. Tierfutterzusammensetzung nach Anspruch 2, wobei die lipophile Matrix 50-100 Gew.-% einer oder mehrerer lipophiler Komponenten, ausgewählt aus Triglyceriden, Diglyceriden, Monoglyceriden, Estern von Monoglyceriden, Estern von Diglyceriden, Phospholipiden, Fettsäureestern von Zuckern und Wachsen, enthält.

4. Tierfutterzusammensetzung nach Anspruch 2 oder 3, wobei die lipophile Matrix einen Schmelzpunkt von mindestens 40 °C hat.

5. Tierfutterzusammensetzung nach einem der vorangehenden Ansprüche, wobei das verkapselte GDL 10-90 Gew.-% der Verkapselungsmatrix umfasst.

6. Tierfutterzusammensetzung nach einem der vorangehenden Ansprüche, wobei GDL 20-90 Gew.-% des verkapselten GDL ausmacht.

7. Tierfutterzusammensetzung nach einem der vorangehenden Ansprüche, wobei mindestens 80 Gew.-% des verkapselten GDL eine Teilchengröße im Bereich von 200-2000 µm aufweisen.

8. Tierfutterzusammensetzung nach einem der vorangehenden Ansprüche, wobei das verkapselte GDL eine Dichte von 0,8-1,4 g/ml aufweist.

9. Tierfutterzusammensetzung nach einem der vorangehenden Ansprüche, wobei die Futterzusammensetzung 0,05-2,0 Gew.-% GDL enthält.

10. Tierfutterzusammensetzung nach einem der vorangehenden Ansprüche, wobei die Zusammensetzung, bezogen auf das Gewicht der Trockenmasse, umfasst:
• 5-30 % Fett;
• 10-30 % Eiweiß;
• 20-65 % Kohlenhydrat;
• 2-15 % Mineralstoffe.

11. Tierfutterzusammensetzung nach einem der vorangehenden Ansprüche, wobei die Zusammensetzung ein Pulver ist.

12. Tierfutterzusammensetzung nach Anspruch 11, wobei die Tierfutterzusammensetzung eine Schüttdichte von 0,4-1,0 g/ml aufweist.

13. Tierfutterzusammensetzung nach einem der vorangehenden Ansprüche zur Verwendung bei der therapeutischen oder prophylaktischen Behandlung von Diarrhoe bei Kälbern oder Ferkeln.

## Revendications

1. Composition d'alimentation animale comprenant 0,05 à 8,0 % en poids, sur la base de la composition totale, de produit d'encapsulation contenant de la glucono-deltalactone (GDL), dans laquelle la GDL est encapsulée dans une matrice d'encapsulation résistant à l'eau et dans laquelle la composition est un substitut de lait de veau ou un substitut de lait de truie.

2. Composition d'alimentation animale selon la revendication 1, dans laquelle la GDL est encapsulée dans une matrice lipophile.

3. Composition d'alimentation animale selon la revendication 2, dans laquelle la matrice lipophile contient 50 à 100 % en poids d'un ou plusieurs composants lipophiles choisis parmi des triglycérides, des diglycérides, des monoglycérides, des esters de monoglycérides, des esters de diglycérides, des phospholipides, des esters d'acide gras de sucres et de cires.

4. Composition d'alimentation animale selon la revendication 2 ou 3, dans laquelle la matrice lipophile a un point de fusion d'au moins 40°C.

5. Composition d'alimentation animale selon l'une quelconque des revendications précédentes, dans laquelle la GDL encapsulée comprend 10 à 90 % en poids de matrice d'encapsulation.

6. Composition d'alimentation animale selon l'une quelconque des revendications précédentes, dans laquelle la GDL représente 20 à 90 % en poids de la GDL encapsulée.

7. Composition d'alimentation animale selon l'une quelconque des revendications précédentes, dans laquelle au moins 80 % en poids de la GDL encapsulée a une taille de particule dans la plage de 200 à 2 000 µm.

8. Composition d'alimentation animale selon l'une quelconque des revendications précédentes, dans laquelle la GDL encapsulée a une densité de 0,8 à 1,4 g/ml.

9. Composition d'alimentation animale selon l'une quelconque des revendications précédentes, dans laquelle la composition d'alimentation contient 0,05 à 2,0 % en poids de GDL.

10. Composition d'alimentation animale selon l'une quelconque des revendications précédentes, dans laquelle la composition comprend, par rapport au poids de matière sèche :
• 5 à 30 % de matières grasses ;
• 10 à 30 % de protéines ;
• 20 à 65 % de glucides ;
• 1 à 15 % de minéraux.

11. Composition d'alimentation animale selon l'une quelconque des revendications précédentes, dans laquelle la composition est une poudre.

12. Composition d'alimentation animale selon la revendication 11, dans laquelle la composition d'alimentation animale a une densité apparente de 0,4 à 1,0 g/ml.

13. Composition d'alimentation animale selon l'une quelconque des revendications précédentes, destinée à être utilisée dans un traitement thérapeutique ou prophylactique de la diarrhée chez des veaux ou des porcelets.
